(19) Europäisches Patentamt
European Patent Office
Office européen des brevets

(11) Veröffentlichungsnummer: **0 008 651**
**B1**

# (12) EUROPÄISCHE PATENTSCHRIFT

(45) Veröffentlichungstag der Patentschrift: **14.10.81**

(51) Int. Cl.³: **C 07 D 249/08**, A 01 N 43/64

(21) Anmeldenummer: **79102560.4**

(22) Anmeldetag: **20.07.79**

## (54) Triazolderivate, Verfahren zu ihrer Herstellung sowie insektizide Mittel, welche diese enthalten.

(30) Priorität: **28.07.78 DE 2833194**

(43) Veröffentlichungstag der Anmeldung: **19.03.80 Patentblatt 80/6**

(45) Bekanntmachung des Hinweises auf die Patenterteilung: **14.10.81 Patentblatt 81/41**

(84) Benannte Vertragsstaaten: **BE CH DE FR GB IT LU NL SE**

(56) Entgegenhaltungen:
**DD-A-111 074**
**DD-A-117 460**
**DE-A-2 725 214**
**DE-A-2 737 489**
**US-A-3 974 174**

(73) Patentinhaber: **BASF Aktiengesellschaft, Carl-Bosch-Strasse 38, D-6700 Ludwigshafen (DE)**

(72) Erfinder: **Linhart, Friedrich, Dr. Chem., Leisberg 61, D-6900 Heidelberg (DE)**
Erfinder: **Zeeh, Bernd, Dr., Thorwaldsenstrasse 5, D-6700 Ludwigshafen (DE)**
Erfinder: **Adolphi, Heinrich, Dr., Kalmitweg 11, D-6703 Limburgerhof (DE)**

**EP 0 008 651 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Triazolderivate, Verfahren zu ihrer Herstellung sowie insektizide Mittel, welche diese enthalten

Die Erfindung betrifft neue Triazolderivate und ihre Herstellung sowie insektizide Mittel, die insektizide Wirkstoffe aus der Gruppe der Carbamate, Phosphor(phosphon)-(di)-(thio)-säureester, Pyrethrine, Pyrethroide, α-Alkyl-phenylessigsäureester, α-Cyclopropyl-phenylessigsäureester und der chlorierten Kohlenwasserstoffe und zur Steigerung der insektiziden Wirksamkeit dieser Wirkstoffe bestimmte Triazolderivate enthalten.

Es ist bekannt, daß beim Einsatz chemischer Wirkstoffe zur Bekämpfung schädlicher Organismen in einigen Fällen bei Mischungen ein wesentlich größerer Effekt erzielt wird als aus der Addition der Einzelwirkungen zu erwarten war. Derartige Wirkungssteigerungen bezeichnet man als Synergismen oder Synergistenwirkungen. Es ist dabei nicht erforderlich, daß der zugesetzte Synergist allein eine erkennbare Wirkung auf den Zielorganismus ausübt.

Bei der Bekämpfung schädlicher Insekten verwendet man in der Praxis fast ausschließlich Piperonalderivate als Synergisten. Von Bedeutung ist insbesondere das Piperonylbutoxid, welches zur Steigerung der Wirkung von Pyrethrinen eingesetzt wird.

Außerdem ist aus der DE-A-2 547 954 bekannt, daß Triazole, die in 1-Stellung einen 2-Halogen-2-phenyl-äthylrest tragen, fungizid wirksam sind.

Es wurde gefunden, daß Triazolderivate der allgemeinen Formel I

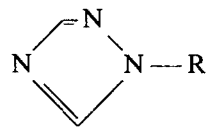 (I)

in der

R einen durch einen unverzweigten oder verzweigten Alkylrest mit 1 bis 4 Kohlenstoffatomen am Phenylring substituierten Benzylrest, einen gegebenenfalls mit bis zu zwei Halogenatomen am Phenylkern substituierten 2-Phenylvinyl-, 3-Phenyl-2-propenyl- oder 2-Phenyl-2-propenylrest, den 1-Phenyläthylrest, den 3-Phenylpropylrest oder den 2-Phenyl-2-chloräthylrest bedeutet, und ihre Salze die Wirksamkeit von insektiziden Wirkstoffen aus der Gruppe der Carbamate, Phosphor(phosphon)-(di)(thio)-säureester, Pyrethrine, Pyrethroide, α-Alkyl-phenyl-essigsäureester, α-Cyclopropylphenyl-essigsäureester und der chlorierten Kohlenwasserstoffe erheblich steigern, so daß insektizide Mittel, die neben diesen Wirkstoffen mindestens ein Triazolderivat der allgemeinen Formel I oder mindestens ein Salz eines Triazolderivats der allgemeinen Formel I enthalten, eine erhebliche größere insektizide Wirksamkeit haben als insektizide Mittel, die die Einzelkomponenten enthalten.

Die Triazolderivate der allgemeinen Formel I und ihre Salze sind sehr stark und breit wirksame Synergisten. Neben einer besonders starken synergistischen Wirkung auf Carbamate steigern sie die Wirkung von Insektiziden aus der Gruppe der Phosphor- bzw. Phosphonsäureester und der entsprechenden Thio- bzw. Dithioester, der Pyrethrine, der Pyrethroide, der α-Alkyl-phenyl-essigsäureester, der α-Cyclopropylphenyl-essigsäureester und der chlorierten Kohlenwasserstoffe.

Als synergistisch wirkende Triazolderivate der allgemeinen Formel I kommen 1,2,4-Triazole oder deren Salze in Betracht, die in 1-Stellung einen Benzylrest der am Phenylring durch einen unverzweigten oder verzweigten Alkylrest mit 1 bis 4 Kohlenstoffatomen, vorzugsweise in 4-Stellung, substituiert ist, den 1-Phenyläthylrest, den 2-Phenyl-2-chloräthylrest, den 3-Phenylpropylrest oder den 2-Phenylvinyl-, 3-Phenyl-2-propenyl- oder 2-Phenyl-2-propenylrest tragen. Der 2-Phenylvinyl-, der 3-Phenyl-2-propenyl- und der 2-Phenyl-2-propenylrest können am Phenylkern bis zu zwei Halogensubstituenten tragen, vorzugsweise Chlor oder Fluor, insbesondere Chlor.

Die Triazolderivate der allgemeinen Formel I, mit Ausnahme der Verbindungen, die als R einen 2-Phenyl-2-chlor-äthylrest tragen, sind neu.

Als Salze kommen Salze der Triazolderivate der allgemeinen Formel I mit anorganischen oder organischen Säuren in Betracht. Beispiele für solche Säuren sind Halogenwasserstoffsäuren, insbesondere Chlorwasserstoff, Bromwasserstoffsäure, Salpetersäure, Schwefelsäure, Oxalsäure, Trichloressigsäure, Aryl- oder Alkylsulfonsäuren, insbesondere p-Toluolsulfonsäure oder Dodecylsulfonsäure.

Beispiele für als Synergisten geeignete Triazolderivate sind 1-(4-Methylbenzyl)-1,2,4-triazol, 1-(4-Äthylbenzyl)-1,2,4-triazol, 1-(4-tert.-Butylbenzyl)-1,2,4-triazol, 1-(3-Phenylpropyl)-1,2,4-triazol, 1-(1-Phenyläthyl)-1,2,4-triazol, 1-(3-Phenyl-2-propenyl)-1,2,4-triazol, 1-(2-Phenyl-2-propenyl)-1,2,4-triazol, 1-(2-Phenyl-2-chloräthyl)-1,2,4-triazol, 1-[2-(2,4-Dichlorphenyl)-vinyl]-1,2,4-triazol, 1-[2-(4-Fluorphenyl)-vinyl]-1,2,4-triazol oder Salze, insbesondere Hydrochloride, dieser Verbindungen.

Triazolderivate der allgemeinen Formel I, in der R einen durch einen unverzweigten oder verzweigten Alkylrest mit 1 bis 4 Kohlenstoffatomen am Phenylring substituierten Benzylrest, einen gegebenenfalls mit bis zu zwei Halogenatomen am Phenylring substituierten 3-Phenyl-2-propenyl- oder 2-Phenyl-2-propenylrest, den 1-Phenyläthylrest oder den 3-Phenylpropylrest bedeutet, werden in an sich bekannter Weise durch Umsetzung von 1,2,4-Triazol mit den entsprechenden Halogeniden der

allgemeinen Formel R—Hal, in der Hal für Halogen steht und R die oben genannten Bedeutungen hat, erhalten. Die Umsetzung wird in Gegenwart eines säurebindenden Mittels, beispielsweise einer organischen Base, wie Triäthylamin, N,N-Dimethylanilin, oder einer anorganischen Base, beispielsweise eines Alkalimetallcarbonats oder -hydrogencarbonats wie Natriumcarbonat oder -hydrogencarbonat, Kaliumcarbonat oder -hydrogencarbonat, oder eines Erdalkalimetallhydroxids und in Gegenwart eines Lösungs- oder Verdünnungsmittels durchgeführt. Geeignete Lösungs- oder Verdünnungsmittels sind Ester, wie Essigester; Alkohole, wie Methanol, Äthanol, Isobutanol, Glykol; Ketone, wie Aceton, Cyclohexanon; Äther, wie Tetrahydrofuran, Dioxan; Toluol, Acetonitril, Dimethylformamid oder Wasser.

Triazolderivate der allgemeinen Formel I, in der R einen gegebenenfalls am Phenylring mit bis zu zwei Halogenatomen substituierten 2-Phenylvinylrest bedeutet, erhält man, wenn man Verbindungen der allgemeinen Formel II

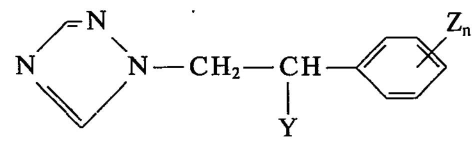

(II)

in der Y und Z für Halogen, insbesondere Chlor, stehen und n 0, 1 oder 2 bedeutet, mit einem Dehydrohalogenierungsmittel, vorzugsweise einer starken Base, in Gegenwart eines Lösungs- oder Verdünnungsmittels umsetzt. Als Basen kommen Hydroxide der Alkalimetalle, wie Natrium- oder Kaliumhydroxid, Alkoholate der Alkalimetalle oder der Erdalkalimetalle, wie Natriummethylat, Magnesiummethylat, Kalium-tert.-butylat oder organische Basen, wie Triäthylamin, in Betracht. Geeignete Lösungs- oder Verdünnungsmittel sind außer Wasser insbesondere Alkohole, wie Methanol; Äther, wie Tetrahydrofuran, Dioxan; Methylglykol, und aprotisch-dipolare Lösungsmittel, wie Dimethylformamid, Acetonitril, N-Methylpyrrolidon, Dimethylsulfoxid.

Triazolderivate der allgemeinen Formel I, in der R einen substituierten 2-Phenylvinylrest bedeutet, werden auch durch Abspaltung von Wasser bzw. Säure aus Verbindungen der allgemeinen Formel II, in der Y eine Hydroxylgruppe oder eine veresterte Hydroxylgruppe bedeutet, erhalten. Die Wasser- bzw. Säureabspaltung wird zweckmäßigerweise mit Hilfe einer starken Säure oder einer Base durchgeführt. Hierfür geeignet sind beispielsweise anorganische Säuren, wie Schwefelsäure, Salzsäure, Phosphorsäure, Aryl- oder Alkylsulfonsäuren, wie p-Toluolsulfonsäure, sowie Alkoholate, wie Erdalkalimetall- oder Alkalimetallalkoholate.

Verbindungen der allgemeinen Formel II, in der Y für eine Hydroxylgruppe und Z für Halogen stehen und n 0, 1 oder 2 bdeutet, werden durch eine für Ketone übliche Reduktionsmethode aus den entsprechenden Triazolylalkanonen der allgemeinen Formel III

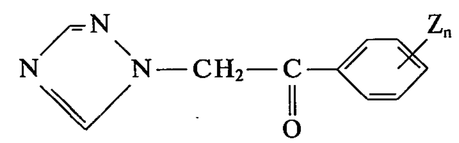

(III)

in der Z und n die oben genannten Bedeutungen haben, hergestellt werden. Die Verbindungen der allgemeinen Formel III sind bekannt und werden durch Umsetzung von 1,2,4-Triazol mit Verbindungen der allgemeinen Formel IV

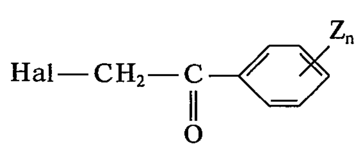

(IV)

in der Z und n die oben genannten Bedeutungen haben und Hal für Chlor oder Brom steht, erhalten (vgl. DE-OS 24 31 407).

Die folgenden Beispiele erläutern die Herstellung der Triazolderivate.

## Beispiel 1

30 Gewichtsteile 1-Phenacyl-1,2,4-triazol werden in 300 Gewichtsteilen Methanol gelöst; dann werden bei 5 bis 10°C 5 Gewichtsteile Natriumborhydrid portionsweise zugegeben. Nach Erwärmen auf Raumtemperatur gibt man etwa 20 Gewichtsteile Ammoniumchlorid zu, säuert mit verdünnter Salzsäure an, erhitzt zum Sieden, stellt mit Ammoniak alkalisch und dampft ein. Der feste Rückstand

wird mit Wasser aufgeschlämmt und ergibt nach dem Absaugen 27,5 Gewichtsteile 1-[2-Phenyl-2-hydroxy-äthyl]-1,2,4-triazol.

Zu 25 Gewichtsteilen 1-[2-Phenyl-2-hydroxyäthyl]-1,2,4-triazol, 300 Gewichtsteilen Chloroform und 1 Gewichtsteil Dimethylformamid tropft man 31 Gewichtsteile Thionylchlorid und erhitzt bis zur Beendigung der Gasentwicklung unter Rückfluß. Nach dem Abkühlen saugt man ab, wäscht den Rückstand mit wenig Chloroform und kristallisiert aus Isopropanol um. Man erhält 22,5 Gewichtsteile 1-[2-Phenyl-2-chloräthyl]-1,2,4-triazolhydrochlorid vom Schmelzpunkt 178° C.

### Beispiel 2

12,5 Gewichtsteile 1-(2-Phenyl-2-chloräthyl)-1,2,4-triazolhydrochlorid werden in Methylenchlorid suspendiert und mit verdünntem Ammoniak verrührt. Die Methylenchloridphase wird abgetrennt, getrocknet und eingedampft, wobei 9,6 Teile 1-(2-Phenyl-2-chloräthyl)-1,2,4-triazol in Form eines Öls mit folgender Analyse zurückbleiben.

| | | | |
|---|---|---|---|
| ber.: | C 57,8 | H 4,9 | N 20,2 |
| gef.: | C 57,8 | H 5,1 | N 20,4. |

### Beispiel 3

15,6 Gewichtsteile 1-[2-(2,4-Dichlorphenyl)-2-chloräthyl]-1,2,4-triazolhydrochlorid werden in 100 Gewichtsteilen Methanol gelöst, mit 20 Gewichtsteilen einer 30%igen Natriummethylatlösung versetzt und 5 Stunden unter Rückfluß erhitzt. Man dampft ein, versetzt mit 200 Teilen Wasser und extrahiert mit 200 Teilen Dichlormethan. Der Extrakt wird mit Natriumsulfat getrocknet, eingedampft und liefert nach dem Umkristallisieren aus Tetrachlormethan 7,5 Gewichtsteile 1-[2-(2,4-Dichlorphenyl)-vinyl]-1,2,4-triazol in Form weißer Nadeln vom Schmelzpunkt 146° C.

### Beispiel 4

Eine Mischung von 14 Gewichtsteilen 1,2,4-Triazol, 200 Gewichtsteilen Acetonitril, 32 Gewichtsteilen Kaliumcarbonat und 40 Gewichtsteilen 3-Phenylpropylbromid wird 12 Stunden lang gerührt. Man filtriert ab, engt das Filtrat ein und erhält durch Destillation 25 Gewichtsteile reines 1-(3-Phenylpropyl)-1,2,4-triazol; Kp: 114 bis 116° C/0,013 mbar.

### Beispiel 5

14 Gewichtsteile 1,2,4-Triazol werden in 200 Gewichtsteilen Acetonitril mit 32 Gewichtsteilen Kaliumcarbonat und 37 Gewichtsteilen 4-Methylbenzylbromid 12 Stunden lang gerührt. Der Rückstand wird abgesaugt, eingeengt und in Essigester gelöst. Das beim Einleiten von Chlorwasserstoff in der Essigesterlösung ausfallende 1-(4-Methylbenzyl)-1,2,4-triazolhydrochlorid vom Schmelzpunkt 155° C wird abgesaugt; Ausbeute: 31 Gewichtsteile.

### Beispiel 6

Eine Mischung von 14 Gewichtsteilen 1,2,4-Triazol, 200 Gewichtsteilen Acetonitril, 32 Gewichtsteilen Kaliumcarbonat und 39,2 Gewichtsteilen Cinnamylchlorid wird 12 Stunden lang gerührt. Man filtriert ab, engt das Filtrat ein, löst es in Dichlormethan, wäscht mit Wasser, dampft ein und destilliert. Man erhält 26 Gewichtsteile 1-(3-Phenyl-2-propenyl)-1,2,4-triazol, Kp: 130 bis 134° C/0,27 mbar.

Analog werden hergestellt

| | |
|---|---|
| 1-(4-Äthylbenzyl)-1,2,4-triazol; | Kp: 122° C/0,27 mbar |
| 1-(4-tert.-Butylbenzyl)-1,2,4-triazol; | Fp: 35° C |
| 1-(1-Phenyläthyl)-1,2,4-triazol; | Kp: 101—102° C/0,13 mbar |
| 1-(2-Phenyl-2-propenyl)-1,2,4-triazol; | Kp: 125—129° C/0,13 mbar |
| 1-[2-(4-Fluorphenyl)-vinyl]-1,2,4-triazolhydronitrat; | Fp: 155° C |

Beispiele für insektizide Wirkstoffe, deren Wirksamkeit sich durch die Triazolderivate der allgemeinen Formel I steigern läßt, sind Carbamate, wie Aryl-N-methylcarbamate, N,N-Dimethylcar-

bamate von Hydroxyheterocyclen oder von Enolen, Oximcarbamate, beispielsweise

1-Naphthyl-N-methyl-carbamat, m-Tolyl-N-methylcarbamat,
2-Isopropyl-phenyl-N-methylcarbamat,
2-sec.-Butyl-phenyl-N-methylcarbamat, 3-(1-Methylbutyl)-phenyl-N-methylcarbamat,
o-Chlorphenyl-N-methylcarbamat, 3,4-Dimethyl-phenyl-N-methylcarbamat,
3,5-Diäthyl-phenyl-N-methylcarbamat, 3-Isopropyl-5-methyl-phenyl-N-methylcarbamat,
6-Chlor-3,4-xylyl-N-methylcarbamat, 3,5-Di-tert.-butyl-phenyl-N-methylcarbamat,
3,4,5-Trimethylphenyl-N-methylcarbamat, 2-Isopropyl-phenyl-N-methylcarbamat,
3,5-Dimethyl-4-methylmercapto-phenyl-N-methylcarbamat,
4-Dimethylamino-m-tolyl-N-methylcarbamat, 4-Dimethylamino-3,5-xylyl-N-methylcarbamat,
4-Diallylamino-3,5-dimethyl-phenyl-N-methylcarbamat,
2-(1,3-Dioxolan-2-yl)-phenyl-N-methylcarbamat, 4-Benzothienyl-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
1-Isopropyl-3-methyl-5-pyrazolyl-N,N-dimethylcarbamat,
2-Dimethyl-carbamoyl-3-methyl-pyrazolyl-(5)-N,N-dimethylcarbamat,
2-Dimethyl-amino-5,6-dimethyl-4-pyrimidinyl-N,N-dimethylcarbamat,
2-(1-Methoxy-2-chlor)-äthoxy-phenyl-N-methylcarbamat,
3-{[(Dimethylamino)-carbonyl]oxy}-1,4-dimethyl-4-propyl-2-pyrazin-5-on,
2-Methyl-2-(methylthio)-propionaldehyd-0-(methylcarbamoyl)-oxim,
S-Methyl-N-[(methylcarbamoyl)oxy]-thioacetimidat,
S-2-Cyanäthyl-N-[(methylcarbamoyl)oxy]-thioacetimidat,
Methyl-N',N'-dimethyl-N-[(methylcarbamoyl)oxy]-1-thiooxamimidat,
2,4-Dimethyl-1,3-dithiolan-2-carboxaldehyd-0-(methylcarbamoyl)-oxim,
3-(Dimethyl-aminomethylen-imino)-phenyl-N-methylcarbamat,
4-(Dimethylamino-methylen-imino)-m-tolyl-N-methylcarbamat,
2-Äthylthiomethyl-phenyl-N-methylcarbamat;

Phosphor- bzw. Phosphonsäureester und die entsprechenden Thio- bzw. Dithioester, beispielsweise

O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyäthyl-carbamoyl-methyl)-phosphordithioat,
O,O-Dimethyl-S-(N-formyl-N-methyl-carbamoyl-methyl)-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diäthyl-carbamoyl)-vinyl]-phosphat,
O,O-Dimethyl-S-(äthylthio)-methyl-phosphordithioat,
O,O-Diäthyl-S-[(p-chlorphenylthio)-methyl]-phosphordithioat,
O,O-Dimethyl-S-(2-äthylthioäthyl)-phosphorthioat,
O,O-Dimethyl-S-(2-äthylthioäthyl)-phosphordithioat,
O,O-Dimethyl-S-(2-äthylsulfinyl-äthyl)-phosphorthioat,
O,O-Diäthyl-S-(2-äthyl-thioäthyl)-phosphordithioat,
O,O-Diäthyl-thiophosphoryl-iminophenyl-acetonitril,
O,O-Diäthyl-S-(2-chlor-1-phthalimidoäthyl)-phosphordithioat,
O,O-Diäthyl-S-[6-chlor-benzoxazolon-(2)-yl(3)]-methyldithiophosphat,
O,O-Dimethyl-S-[2-methoxy-1,3,4-thiodiazol-5-[4H]-onyl(4)-methyl]-phosphordithioat,
O,O-Diäthyl-O-[3,5,6-trichlorpyridyl-(2)]-phosphorthioat,
O,O-Diäthyl-O-(2-pyrazinyl)-phosphorthioat,
O,O-Diäthyl-O-[2-isopropyl-4-methylpyridinyl(6)]-phosphorthioat,
O,O-Diäthyl-O-[(2-diäthylamino)-6-methyl-4-pyrimidinyl]-thionophosphat,
O,O-Dimethyl-S-(4-oxo-1,2,3-benzotriazin-3-yl-methyl)-phosphordithioat,
O,O-Dimethyl-S-[(4,6-diamino-1,3,5-triazin-2-yl)-methyl]-phosphordithioat,
O,O-Diäthyl-(1-phenyl-1,2,4-triazol-3-yl)-thionophospat,
O,S-Dimethyl-phosphoramidothioat,
O,S-Dimethyl-N-acetyl-phosphoramidothioat,
O,O-Dimethyl-O-(p-nitrophenyl)-phosphorthioat,
O,O-Diäthyl-O-(p-nitrophenyl)-phosphorthioat,
O-Äthyl-O-(p-nitrophenyl)-phenyl-phosphonothioat,
O,O-Dimethyl-O-(3-methyl-4-nitrophenyl)-phosphorthioat,
O,O-Diäthyl-O-(2,4-dichlorphenyl)-phosphorthioat,
O-Äthyl-O-(2,4-dichlorphenyl)-phenylphosphonothioat,
O,O-Dimethyl-O-(2,4,5-trichlorphenyl)-phosphorthioat,

O-Äthyl-O-(2,4,5-trichlorphenyl)-äthylphosphonothioat,
O,O-Dimethyl-O-(4-brom-2,5-dichlorphenyl)-phosphorthioat,
O,O-Dimethyl-O-(2,5-dichlor-4-jodphenyl)-phorphorthioat,
O,O-Dimethyl-O-(3-methylthiophenyl)-phosphorthioat,
O-Äthyl-O-(3-methyl-4-methylthiophenyl)-isopropyl-phosphoramidat,
O,O-Diäthyl-O-[p-(methylsulfinyl)-phenyl]-phosphorthioat,
O-Äthyl-S-phenyl-äthyl-phosphonodithioat,
O,O-Diäthyl-[2-chlor-1-(2,4-dichlorphenyl)-vinyl]-phosphat,
O,O-Dimethyl-[2-chlor-1-(2,4,5-trichlorphenyl)]-vinyl-phosphat,
O,O-Dimethyl-S-(1-phenyl)-äthylacetat-phosphordithioat,
Bis-(dimethylamino)-fluor-phosphinoxid, Octamethyl-pyrophosphoramid,
O,O,O,O-Tetraäthyldithio-pyrophosphat, S-Chlormethyl-O,O-diäthyl-phosphordithioat,
O-Äthyl-S,S-dipropyl-phosphordithioat, O,O-Dimethyl-O-2,2-dichlorvinyl-phosphat,
O,O-Dimethyl-1,2-dibrom-2,2-dichloräthyl-phosphat,
O,O-Dimethyl-2,2,2-trichlor-1-hydroxy-äthyl-phosphonat,
O,O-Dimethyl-S-[1,2-biscarbäthoxy-äthyl(1)]-phosphordithioat;

Pyrethrine, beispielsweise ein Wirkstoffgemische aus Pyrethrin I, Pyrethrin II, Cinerin I und Cinerin II aus Chrysanthemum-cinerae-folium-Blüten;
synthetische Pyrethroide, wie Ester der 2,2-Dimethyl-3-(2,2-dimethylvinyl)-cyclopropancarbonsäure, beispielsweise

2-Allyl-3-methylcyclopenten-(2)-on-1-yl-(4)-chrysanthemat,
3,4,5,6-Tetrahydro-phthalimidomethyl-DL-cis,transchrysanthemat,
5-Benzyl-furyl-(3)-methyl-DL-cis,transchrysanthemat,
2-Methyl-5-(2-propinyl)-3-furylmethylchrysanthemat oder Ester der
2,2-Dimethyl-3-(2,2-dihalogenvinyl)-cyclopropancarbonsäure, beispielsweise
3-Phenoxybenzyl(±)-cis,trans-2,2-dimethyl-3-(2,2-dichlorvinyl)-cyclopropan-1-carboxylat,
α-Cyano-3-phenoxybenzyl(±)-cis,trans-2,2-dimethyl-3-(2,2-dichlorvinyl)-cyclopropancarboxylat,
(s)-α-Cyano-3-phenoxybenzyl-cis(1R, 3R)-2,2-dimethyl-3-(2,2-dibromvinyl)-cyclopropancarboxylat;

α-Alkyl-phenylessigsäureester, beispielsweise α-Isopropylphenyl-essigsäureester, insbesondere

α-Cyano-3-phenoxybenzyl-α-isopropyl-4-chlorphenylacetat,
3-Phenoxybenzyl-α-isopropyl-4-chlor-phenylacetat,
5-Benzyl-3-furylmethyl-α-isopropyl-4-chlorphenylacetat,
5-Benzyl-3-furylmethyl-α-isopropyl-4-methylphenylacetat,
3-Phenoxybenzyl-α-isopropyl-4-methylphenylacetat,
3-Phenoxybenzyl-α-isopropyl-4-methoxyphenylacetat,
5-Benzyl-3-methylfuryl-α-isopropyl-4-methoxyphenylacetat;

α-Cyclopropyl-phenylessigsäureester, beispielsweise

α-Cyano-3-phenoxybenzyl-α-cyclopropyl-4-chlorphenylacetat,
5-Benzyl-3-furylmethyl-α-cyclopropyl-4-chlorphenylacetat,
α-Cyano-3-phenoxybenzyl-α-cyclopropyl-4-bromphenylacetat;

und chlorierte Kohlenwasserstoffe, beispielsweise

γ-Hexachlorcyclohexan, 1,1-Di-(p-methoxyphenyl)-2,2,2-trichloräthan,
6,7,8,9,10,10-Hexachlor-1,5,5a,6,9,9a-hexahydro-6,9-methano-2,4,3-benzodioxathiepin-3-oxid.

Die Triazolderivate oder ihre Salze und die insektiziden Wirkstoffe können in den erfindungsgemäßen Mitteln in einem verhältnismäßig breiten Mengenverhältnis eingesetzt werden. In den seltensten Fällen dürfte aber ein Gewichtsverhältnis, das über den Bereich 1 : 10 bis 10 : 1 hinausgeht, sinnvoll sein. Bevorzugt ist ein Gewichtsverhältnis im Bereich von 1 : 5 bis 5 : 1.

Die erfindungsgemäßen insektiziden Mittel können gegenüber einer Vielzahl von Pflanzen- und Haushaltsschädlingen angewendet werden. Sie können als solche, in Form ihrer Formulierungen oder den daraus bereiteten Anwendungsformen, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich nach den Verwendungszwecken. Die Triazolderivate und die

insektiziden Wirkstoffe bzw. ihre Formulierungen können dabei sowohl gemeinsam als auch getrennt ausgebracht werden.

Die Formulierungen enthalten im allgemeinen zwischen 0,1 und 95 Gew.-% Synergist und Wirkstoff, vorzugsweise zwischen 0,5 und 90 Gew.-%.

Die Konzentrationen von Synergist und Wirkstoff in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit guten Erfolg im Ultra-Low-Volume-Verfahren (ULV) verwendet werden, wobei es möglich ist, Formulierungen mit einer Konzentration an Synergist und Wirkstoff von über 95 Gew.-% oder sogar eine Mischung aus Synergist und Wirkstoff allein auszubringen.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz sowie Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäuren, Alkylarylsulfonate, Alkylsulfate, Alkylsulfonate, Alkali- und Erdalkalisalze der Dibutylnaphthalinsulfonsäure, Laurylläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfiniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenolpolyglykoläther, Alkylarylpolyätheralkohole, Isotridecylalkohol, Fettalkoholäthylenoxid-Kondensate, äthoxyliertes Rizinusöl, Polyoxyäthylalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreise, Bolus, Löß, Ton, dolomit, Diatomeenerde, Kalcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den erfindungsgemäßen Mitteln können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 :. 10 bis 10 : 1 zugemischt werden.

Die Verwendung der erfindungsgemäßen insektiziden Mittel erleichtert die Bekämpfung von Pflanzen- und Haushaltsschädlingen. Beispiele hierfür sind schädliche Insekten aus der Ordnung der Schmetterlinge (Lepidoptera), z. B. Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cereallella (Getreidemotte), Phthorimaea opercullela (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellinella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia

gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling); aus der Ordnung der Käfer (Coleoptera), z. B. Blitophaga undata (Schwarzer Rübenaaskäfer), Melanotus communis (Drahtwurm), Lumonius californicus (Drahtwurm), Agriotes lineatus (Saatschnellkäfer), Agriotes abscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melontha hippocastani (Waldmaikäfer), Amphimallus solstitialis (Brachkäfer), Crioceris asparagi (Spargelhähnchen), Lema melanopus (Getreidehähnchen), Leptinotars decemlineata (Kartoffelkäfer), Phaedon cochleariae (Meerrettich-Blattkäfer), Phyllotreta nemorum (Kohlerdfloh), Chaetocnema tibialis (Rübenflohkäfer), Psylloides chrysocephala (Raps-Flohkäfer), Diabrotica 12-punctata (Südlicher Maiswurzelwurm), Cassida neblulosa (Nebliger Schildkäfer), Bruchus lentis (Linsenkäfer), Bruchus rufimanus (Pferdebohnenkäfer), Bruchus pisorum (Erbsenkäfer), sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer brauner Rüsselkäfer), Byctiscus betulae (Rbenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);
aus der Ordnung der Zweiflügler (Diptera), z. B. Mayetiola destructor (Hessenfliege), Dasyneura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege);
aus der Ordnung der Hautflügler (Hymenoptera), z. B. Athalia rosae (Rübsenblattwespe), Hoplocampa minuta (Pflaumensägewespe), Monomorium pharaonis (Pharaoameise), Solenopsis geminata (Feuerameise), Atta sexdens (Blattschneiderameise);
aus der Ordnung der Wanzen (Heteroptere), z. B. Nezara viridula (Grüne Reiswanze), Eurygaster integriceps (Asiatische Getreidewanze), Blissus leucopterus (Chinch bug.), Dysdercus cingulatus (Kapok-Wanze), Piesma quadrata (Rübenwanze), Lygus pratensis (Gemeine Wiesenwanze);
aus der Ordnung der Planzensauger (Homoptera), z. B. Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Sysaulocorthum pseudosolani (Gefleckte Kartoffellaus), Acrythosiphon onobrychis (Grüne Erbsenlaus), Macrosiphon rosae (Große Rosenblattlaus), Megoura viciae (Wickenlaus);
und die zu der Klasse der Arachnoidea gehörenden Milben und Zecken (Acarina), z. B. Tetranychus urticae, Tetranychus atlanticus, Tetranychus pacificus, Paratetranychus pilosus, Bryobia praetiosa, Ixodes ricinus (Holzbock), Ornithodorus moubata, Ablyomma americanum, Dermacentor silvarum, Boophilus microplus.

Die folgenden biologischen Versuche belegen die Synergistenwirkung. Sie sind jedoch nur als Beispiele zu betrachten und umreißen in keinem Falle den ganzen erfaßten Bereich.

In den Tabellen bedeuten die Synergisten

Nr. 1

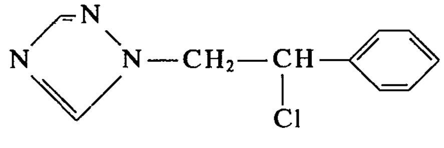

Nr. 2

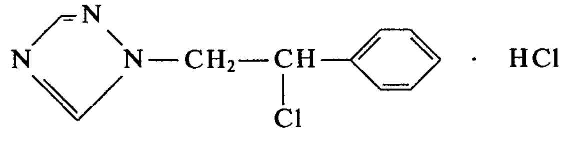

Nr. 3

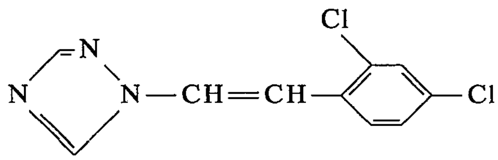

Nr. 4

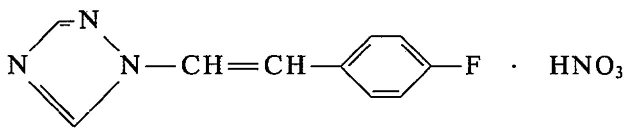

Nr. 5

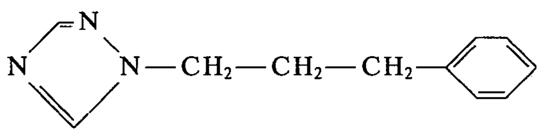

Nr. 6

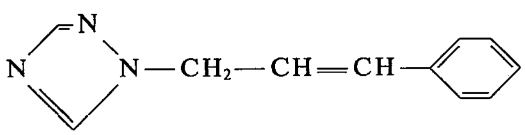

Nr. 7

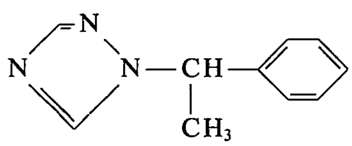

Nr. 8

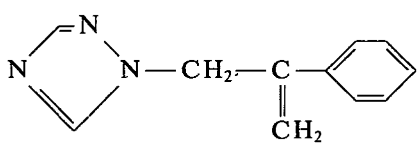

Nr. 9

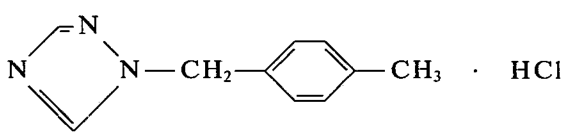

Nr. 10

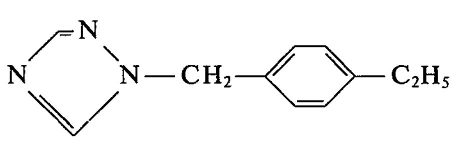

Nr. 11

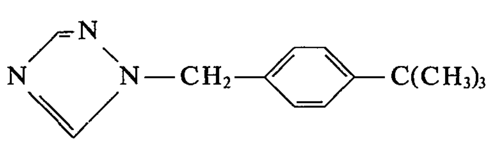

Als insektizide Wirkstoffe werden 1-Naphthyl-N-methylcarbamat (Carbaryl), 2,5-Diäthylphenyl-N-methylcarbamat (Fenethcarb), 2-Dimethylamino-5,6-dimethyl-4-pyrimidinyl-N,N-dimethyl-carbamat (Pirimicarb), O,O-Dimethyl-S-[1,2bis-carbäthoxy-äthyl-(1)]-phosphordithioat (Malathion), DL-2-Allyl-3-methyl-cyclopenten-(2)-on-(1)-yl-(4)-DL-cis,trans-chrysanthemat (Allethrin), und ein Wirkstoffgemisch, bestehend aus Pyrethrin I, Pyrethrin II, Cinerin I und Cinerin II aus Chrysanthemum cineraefolium Blüten (Pyrethrine) verwendet.

## Beispiel A

### Synergistenwirkung bei Schaben (Blatta orientalis)

Der Boden eines 1-l-Glases wird mit der acetonischen Lösung des Insektizides und des Synergisten in den angegebenen Mengenverhältnissen behandelt. Nach Verdunsten des Lösungsmittels belegt man die Gläser mit je 5 ausgewachsenen Schaben (Blatta orientalis) und bestimmt nach 48 Stunden die Mortalitätsrate. Die Aufwandmengen liegen in logarithmischer Stufung und erlauben die Erstellung einer Dosis-Effekt-Kurve. Die LD 50 wird graphisch ermittelt und dient als Grundlage der Bewertung.

Als Insektizide werden Carbaryl, Fenethcarb, Pirimicarb, Malathion und Allethrin eingesetzt; das Verhältnis Insektizid: Synergist beträgt 1 : 5 Gewichtsteile.

| Wirkstoff | LD 50 |
|---|---|
| Nr. 1 | >5,0 mg |
| Nr. 2 | >5,0 mg |
| Nr. 3 | >5,0 mg |
| Nr. 4 | >5,0 mg |
| Nr. 5 | >2,0 mg |
| Nr. 6 | 9,0 mg |
| Nr. 7 | >2,0 mg |
| Nr. 8 | 9,0 mg |
| Nr. 9 | >2,0 mg |
| Nr. 10 | >2,0 mg |
| Nr. 11 | >10,0 mg |
| Carbaryl | 0,25 mg |
| Carbaryl + Nr. 1 | 0,25 mg + 1,25 mg |
| Carbaryl + Nr. 2 | 0,026 mg + 0,13 mg |
| Carbaryl + Nr. 3 | 0,04 mg + 0,2 mg |
| Carbaryl + Nr. 4 | 0,02 mg + 0,01 mg |
| Carbaryl + Nr. 5 | 0,015 mg + 0,075 mg |
| Carbaryl + Nr. 7 | 0,012 mg + 0,06 mg |
| Carbaryl + Nr. 9 | 0,018 mg + 0,09 mg |
| Carbaryl + Nr. 10 | 0,015 mg + 0,075 mg |

Fortsetzung

| Wirkstoff | LD 50 |
|---|---|
| Fenethcarb | 0,45 mg |
| Fenethcarb + Nr. 1 | <0,02 mg + 0,1 mg |
| Fenethcarb + Nr. 2 | 0,13 mg + 0,65 mg |
| Fenethcarb + Nr. 3 | 0,035 mg + 0,175 mg |
| Fenethcarb + Nr. 5 | 0,24 mg + 1,2 mg |
| Fenethcarb + Nr. 6 | 0,2 mg + 1,0 mg |
| Fenethcarb + Nr. 7 | 0,11 mg + 0,55 mg |
| Fenethcarb + Nr. 8 | 0,14 mg + 0,7 mg |
| Fenethcarb + Nr. 9 | 0,16 mg + 0,8 mg |
| Fenethcarb + Nr. 10 | 0,24 mg + 1,2 mg |
| Fenethcarb + Nr. 11 | 0,12 mg + 0,6 mg |
| Pirimicarb | 30 mg unwirksam |
| Pirimicarb + Nr. 5 | 0,11 mg + 0,55 mg |
| Pirimicarb + Nr. 6 | 1,2 mg + 6,0 mg |
| Pirimicarb + Nr. 7 | 0,45 mg + 2,25 mg |
| Pirimicarb + Nr. 8 | 0,25 mg + 1,25 mg |
| Pirimicarb + Nr. 10 | 0,65 mg + 3,25 mg |
| Pirimicarb + Nr. 11 | 0,7 mg + 3,5 mg |
| Malathion | 0,12 mg |
| Malathion + Nr. 3 | 0,09 mg + 0,45 mg |
| Malathion + Nr. 7 | 0,09 mg + 0,45 mg |
| Malathion + Nr. 9 | 0,08 mg + 0,4 mg |
| Allethrin | 0,11 mg |
| Allethrin + Nr.1 | 0,021 mg + 0,105 mg |
| Allethrin + Nr. 2 | 0,021 mg + 0,105 mg |
| Allethrin + Nr. 3 | 0,045 mg + 0,225 mg |
| Allethrin + Nr. 5 | 0,034 mg + 0,17 mg |
| Allethrin + Nr. 6 | 0,06 mg + 0,3 mg |
| Allethrin + Nr. 7 | 0,04 mg + 0,2 mg |

Fortsetzung

| Wirkstoff | LD 50 |
| --- | --- |
| Allethrin + Nr. 8 | 0,024 mg + 0,12 mg |
| Allethrin + Nr. 9 | 0,04 mg + 0,2 mg |
| Allethrin + Nr. 10 | 0,04 mg + 0,2 mg |
| Allethrin + Nr. 11 | 0,024 mg + 0,12 mg |

Beispiel B

Synergistenwirkung bei Stubenfliegen (Musca domestica);
Dauerkontakt

Beide Hälften einer Petrischale von 10 cm Durchmesser werden mit insgesamt 2 ml der acetonischen Wirkstofflösungen ausgekleidet. Nach Verdunsten des Lösungsmittels (ca. 30 Minuten) bringt man je 10 Fliegen in die Schalen.

Die Mortalitätsrate wird nach 4 Stunden und die LD 50 graphisch ermittelt.

| Wirkstoff | LD 50 |
| --- | --- |
| Pyrethrine | 0,015 mg |
| Nr. 1 | >2,0 mg |
| Pyrethrine + Nr. 1 | 0,0055 mg + 0,0275 mg |
| Nr. 2 | 0,2 mg |
| Pyrethrine + Nr. 2 | 0,006 mg + 0,03 mg |
| Nr. 4 | >2,0 mg |
| Pyrethrine + Nr. 4 | 0,005 mg + 0,025 mg |

Beispiel C

Synergistenwirkung bei Stubenfliegen (Musca domestica);
Applikationstest

Verwendet werden adulte Stubenfliegen (4 Tage nach dem Schlüpfen). Diese Tiere erhalten in leichter $CO_2$-Narkose je 1 µl der acetonischen Wirkstofflösung auf das ventrale Abdomen appliziert.

20 Fliegen mit gleicher Behandlung bringt man in einen Cellophanbeutel (Volumen ca. 500 ml). Nach 4 Stunden ermittelt man die Mortalitätsrate, erstellt aus den unterschiedlichen Konzentrationen die Dosis-Mortalitäts-Kurve und ermittelt die LD 50.

| Wirkstoff | LD 50 |
| --- | --- |
| Pyrethrine | 0,1 µg/Fliege |
| Nr. 5 | 10 µg/Fliege unwirksam |
| Pyrethrine + Nr. 5 | 0,035 + 0,35 µg/Fliege |

Beispiel D

Synergistenwirkung bei Kornkäfer (Sitophilus granaria)

Petrischalen von 10 cm Durchmesser werden mit acetonischer Wirkstofflösung ausgekleidet. Nach Verdunsten des Lösungsmittels belegt man die Schalen mit 50 Kornkäfern.

Nach 4 Stunden werden die Käfer in unbehandelte Gefäße überführt. Die Mortalitätsrate wird nach 24 Stunden ermittelt. Dabei wird festgestellt, wieviele Käfer in der Lage sind, nach diesem Zeitpunkt innerhalb von 60 Minuten ein unbehandeltes Pappschälchen (Durchmesser: 40 mm, Höhe: 10 mm) zu verlassen.

| Wirkstoff | LD 50 |
|---|---|
| Carbaryl | 2,5 mg |
| Nr. 1 | 2 mg unwirksam |
| Carbaryl + Nr. 1 | 0,17 mg + 0,85 mg |
| Nr. 5 | 2 mg unwirksam |
| Carbaryl + Nr. 5 | 0,085 mg + 0,425 mg |
| Nr. 6 | 2 mg unwirksam |
| Carbaryl + Nr. 6 | 1 mg + 1 mg (Mortalitätsrate >95 %) |
| Nr. 8 | 2 mg unwirksam |
| Carbaryl + Nr. 8 | 0,23 mg + 1,15 mg |
| Nr. 9 | 4 mg unwirksam |
| Carbaryl + Nr. 9 | 0,52 mg + 2,6 mg |
| Nr. 10 | 2 mg unwirksam |
| Carbaryl + Nr. 10 | 0,17 mg + 0,85 mg |
| Nr. 11 | 2 mg unwirksam |
| Carbaryl + Nr. 11 | 1 mg + 1 mg (Mortalitätsrate >95 %) |

| Wirkstoff | LD 50 |
|---|---|
| Pyrethrine | 1,2 mg |
| Nr. 1 | 1 mg unwirksam |
| Pyrethrine + Nr. 1 | 0,15 mg + 0,75 mg |
| Nr. 5 | 2 mg unwirksam |
| Pyrethrine + Nr. 5 | 0,07 mg + 0,35 mg |

Beispiele E

Synergistenwirkung auf Stubenfliegen (Musca domestica);
Tauchversuch

100 ml der wäßrigen Wirkstoffaufbereitung (Wirkstoffkonzentration in %) werden über 20 adulte Stubenfliegen (Musca domestica) gegossen, die auf einem Filter in einer Nutsche (∅ 15 cm) liegen. Ein Gazedeckel verhindert das Aufschwimmen der Tiere bei der Behandlung. Nach 5 Sekunden wird die Wirkstoffaufbereitung abgesaugt und die Fliegen in eine saubere Petrischale vom Durchmesser 10 cm überführt, deren Boden mit einem Rundfilter ausgelegt ist.

Nach 4 bzw. 24 Stunden registriert man die Tiere in Rückenlage.

Ergebnis

| Wirkstoff | LD 50 nach 4 Stunden | LD 50 nach 24 Stunden |
|---|---|---|
| Pirimicarb | 0,05 % | 0,04 % |
| Nr. 1 | 0,085 % | 0,05 % |
| Pirimicarb + Nr. 1 | 0,0025 %+ 0,0125 % | <0,0025 %+ 0,0125 % |
| Nr. 2 | 0,2 % unwirksam | 0,1 % |
| Pirimicarb + Nr. 2 | 0.004 %+ 0,02 % | <0,004 %+ 0,02 % |
| Nr. 3 | >0,2 % | 0,05 % |
| Pirimicarb + Nr. 3 | 0,0035 %+ 0,0175 % | 0,0015 %+ 0,0075 % |
| Nr. 5 | >0,2 % | 0,05 % |
| Pirimicarb + Nr. 5 | 0,01 %+ 0,5 % | 0,004 %+ 0,02 % |
| Nr. 6 | >0,2 % | 0,07 % |
| Pirimicarb + Nr. 6 | 0,009 %+ 0,45 % | 0,004 %+ 0,02 % |

**Patentansprüche**

1. Triazolderivate der allgemeinen Formel I

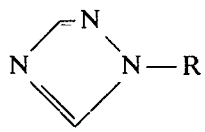

(I)

in der
R einen durch einen unverzweigten oder verzweigten Alkylrest mit 1 bis 4 Kohlenstoffatomen am Phenylring substituierten Benzylrest, einen gegebenenfalls mit bis zu zwei Halogenatomen am Phenylring substituierten 2-Phenylvinyl-, 3-Phenyl-2-propenyl- oder 2-Phenyl-2-propenylrest, den 1-Phenyläthylrest oder den 3-Phenylpropylrest bedeutet, und ihre Salze.

2. 1-(4-tert.-Butylbenzyl)-1,2,4-triazol.

3. Insektizides Mittel, gekennzeichnet durch einen Gehalt an mindestens einem insektiziden Wirkstoff aus der Gruppe der Carbamate, Phosphor(phosphon)-(di)(thio)säureester, Pyrethrine, Pyrethroide, α-Alkyl-phenylessigsäureester, α-Cyclopropyl-phenylessigsäureester und der chlorierten Kohlenwasserstoffe und mindestens einem Triazolderivat der Formel I′

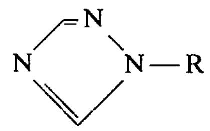

(I′)

in der

R einen durch einen unverzweigten oder verzweigten Alkylrest mit 1 bis 4 Kohlenstoffatomen am Phenylring substiuierten Benzylrest, einen gegebenenfalls mit bis zu zwei Halogenatomen am Phenylring substituierten 2-Phenylvinyl-, 3-Phenyl-2-propenyl- oder 2-Phenyl-2-propenylrest, den 1-Phenyläthylrest, den 3-Phenylpropylrest oder den 2-Phenyl-2-chlor-äthylrest bedeutet, oder einem Salz eines solchen Triazolderivats als Synergisten.

4. Insektizides Mittel nach Anspruch 3, dadurch gekennzeichnet, daß das Gewichtsverhältnis von insektizidem Wirkstoff zu Triazolderivat oder zu einem Salz davon 1 : 10 bis 10 : 1 beträgt.

5. Insektizides Mittel nach Anspruch 3, dadurch gekennzeichnet, daß es als insektiziden Wirkstoff ein Carbamat enthält.

6. Verfahren zur Herstellung von Triazolderivaten der allgemeinen Formel I

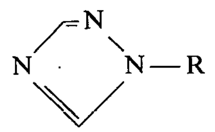

(I)

in der

R einen durch einen unverzweigten oder verzweigten Alkylrest mit 1 bis 4 Kohlenstoffatomen am Phenylring substituierten Benzylrest, einen gegebenenfalls mit bis zu zwei Halogenatomen am Phenylring substituierten 3-Phenyl-2-propenyl- oder 2-Phenyl-2-propenylrest, den 1-Phenyläthylrest oder den 3-Phenylpropylrest bedeutet, dadurch gekennzeichnet, daß man 1,2,4-Triazol mit einem Halogenid der allgemeinen Formel R-Hal, in der Hal für ein Halogenatom steht und R die obengenannten Bedeutungen hat, in Gegenwart eines säurebindenden Mittels und eines Lösungs- oder Verdünnungsmittels in an sich bekannter Weise umsetzt.

7. Verfahren zur Herstellung von Triazolderivaten der allgemeinen Formel I

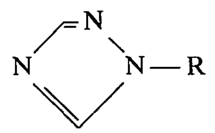

(I)

in der

R einen gegebenenfalls mit bis zu zwei Halogenatomen am Phenylring durch Halogen substituierten 2-Phenylvinylrest bedeutet, dadurch gekennzeichnet, daß man Verbindungen der allgemeinen Formel II

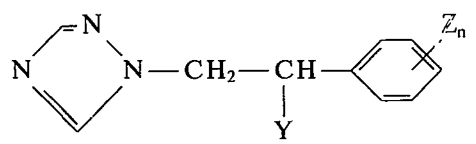

(II)

in der

Y und Z jeweils für ein Halogenatom stehen und n 0,1 oder 2 bedeutet, mit einem Dehydrohalogenierungsmittel in Gegenwart eines Lösungs- oder Verdünnungsmittels in an sich bekannter Weise umsetzt.

**Claims**

1. A triazole derivative of the general formula I

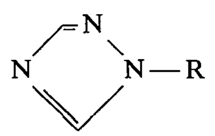

(I)

where R denotes benzyl substituted on the phenyl ring by linear or branched alkyl of 1 to 4 carbon atoms, 2-phenylvinyl which is unsubstituted or substituted by up to two halogens on the phenyl ring, 3-phenyl-2-propenyl which is unsubstituted or substituted by up to two halogens on the phenyl ring, 2-phenyl-2-propenyl which is unsubstituted or substituted by up to two halogens on the phenyl ring, 1-phenylethyl or 3-phenylpropyl, or a salt thereof.

2. 1-(4-tert-butylbenzyl)-1,2,4,triazole.

3. An insecticidal agent characterized by a content of at least one insecticidal active ingredient selected from the group consisting of carbamates, phosphoric (phosphonic) (di) (thio) acid esters, pyrethrins, pyrethroids, α-alkylphenyl acetates, α-cyclopropylphenyl acetates and chlorinated hydrocarbons, and at least one triazole derivative of the formula I'

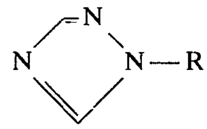
(I')

where R denotes benzyl substituted on the phenyl ring by linear or branched alkyl of 1 to 4 carbon atoms, 2-phenylvinyl which is unsubstituted or substituted by up to two halogens on the phenyl ring, 3-phenyl-2-propenyl which is unsubstituted or substituted by up to two halogens on the phenyl ring, 2-phenyl-2-propenyl which is unsubstituted or substituted by up to two halogens on the phenyl ring, 1-phenylethyl, 3-phenylpropyl or 2-phenyl-2-chloroethyl, or a salt of such a triazole derivative, as synergist.

4. An insecticidal agent as claimed in claim 3, characterized in that the weight ratio of insecticidal active ingredient to triazole derivative or a salt thereof is from 1 : 10 to 10 : 1.

5. An insecticidal agent as claimed in claim 3, characterized in that it contains a carbamate as the insecticidal active ingredient.

6. A process for the production of a triazole derivative of the general formula I

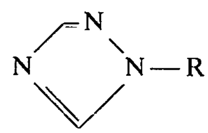
(I)

where R denotes benzyl substituted on the phenyl ring by linear or branched alkyl of 1 to 4 carbon atoms, 3-phenyl-2-propenyl which is unsubstituted or substituted by up to two halogens on the phenyl ring, 2-phenyl-2-propenyl which is unsubstituted or substituted by up to two halogens on the phenyl ring, 1-phenylethyl or 3-phenylpropyl, characterized in that 1,2,4-triazole is reacted in a conventional manner with a halide of the general formula R-Hal, where Hal denotes halogen and R has the above meanings, in the presence of an acid-binding agent and a solvent or diluent.

7. A process for the production of a triazole derivative of the general formula I

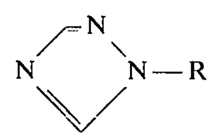
(I)

where R denotes 2-phenylvinyl which is unsubstituted or substituted by up to two halogens on the phenyl ring, characterized in that a compound of the general formula II

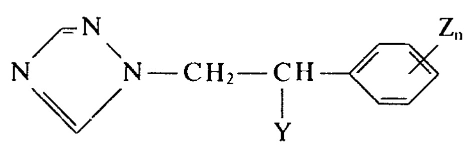
(II)

where Y and Z each denote halogen and n denotes 0,1 or 2, is reacted in a conventional manner with a dehydrohalogenating agent in the presence of a solvent or diluent.

**Revendications**

1. Dérivés de triazole de formule générale I et leurs sels

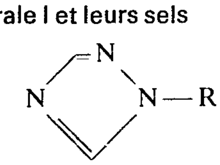
(I)

dans laquelle

R représente un reste benzyle substitué sur le noyau phényle par un reste alkyle en $C_1$ à $C_4$ ramifié ou non, un reste 2-phénylvinyl-3-phényl-2-propényl- ou 2-phényl-2-propenyle éventuellement substitué avec jusqu'à deux atomes d'halogène sur le noyau phényle, le reste 1-phényléthyle ou le reste 3-phénylpropyle.

2. 1-(4-tert.-butylbenzyl)-1,2,4-triazole.

3. Agent insecticide caractérisé par le fait qu'une teneur en au moins un principe actif insecticide du groupe des carbamates, di-thioester d'acide phosphor-(phosphonique), pyréthrine, pyréthroïde, ester d'acide α-alkylphénylacétique, ester d'acide α-cyclopropyl-phénylacétique et des hydrocarbures chlorés et au moins un dérivé de triazole de formule I'

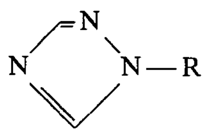

(I')

dans laquelle
R représente un reste benzyle substitué sur le noyau phényle par un reste alkyle en $C_1$ à $C_4$ ramifié ou non, un reste 2-phénylvinyl-3-phényl-2-propényl- ou 2-phényl-2-propényle éventuellement substitué avec jusqu'à deux atomes d'halogène sur le noyau phényle, le reste 1-phényléthyle ou le reste 3-phénylpropyle ou un sel d'un tel dérivé de triazole comme synergiste.

4. Agent insecticide selon la revendication 3, caractérisé par le fait que le rapport en poids du principe actif insecticide au dérivé de triazole ou à son sel est de 1/10 à 10/1.

5. Agent insecticide selon la revendication 3, caractérisé par le fait qu'il contient un carbamate comme principe actif insecticide.

6. Procédé de préparation de dérivés de triazole de formule générale I

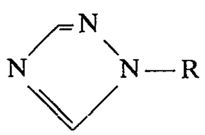

(I)

dans laquelle
R représente un reste benzyle substitué sur le noyau phényle par un reste alkyle en $C_1$ à $C_4$ ramifié ou non, un reste 3-phényl-2-propionyle ou 2-phényl-2-propényle éventuellement substitué avec jusqu'à deux atomes d'halogène sur le noyau phényle, le reste 1-phényléthyle ou le reste 3-phénylpropyle, caractérisé par le fait qu'on fait réagir, de manière connue en soi, du 1,2,4-triazole avec un halogénure de formule générale R-Hal, dans laquelle Hal représente un atome d'halogène et R a les significations indiquées plus haut, en présence d'un agent liant les acides et un solvant ou diluant.

7. Procédé de préparation de dérivés de triazole de formule générale I

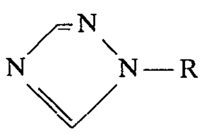

(I)

dans laquelle
R représente un reste 2-phénylvinyle éventuellement substitué avec jusqu'à deux atomes d'halogène sur le noyau phényle, caractérisé par le fait que l'on fait réagir, de manière connue en soi, en présence d'un solvant ou diluant, des composés de formule générale II

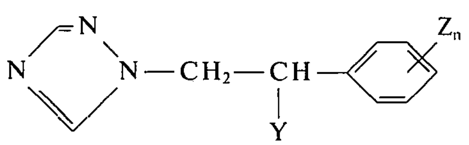

(II)

dans laquelle
Y et Z représentent chacun un atome d'halogène, et n 0,1 ou 2, avec un agent de déhydrohalogénation.